(19) Europäisches Patentamt / European Patent Office / Office européen des brevets

(11) **EP 1 913 933 A1**

(12) **DEMANDE DE BREVET EUROPEEN**

(43) Date de publication:
**23.04.2008 Bulletin 2008/17**

(51) Int Cl.:
*A61K 8/81* (2006.01)     *A61Q 1/04* (2006.01)
*A61Q 1/10* (2006.01)     *A61K 8/04* (2006.01)
*A61K 8/25* (2006.01)     *A61Q 1/00* (2006.01)
*A61Q 1/06* (2006.01)     *A61Q 1/08* (2006.01)

(21) Numéro de dépôt: **07118483.2**

(22) Date de dépôt: **15.10.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **16.10.2006  FR 0654302**
**20.12.2006  FR 0655740**

(71) Demandeur: **L'ORÉAL**
**75008 Paris (FR)**

(72) Inventeurs:
• **Dumousseaux, Christophe**
**TOKYO (JP)**
• **Kawamoto, Makoto**
**KANAGAWA (JP)**

(74) Mandataire: **Tanty, François**
**Nony & Associés,**
**3, rue de Penthièvre**
**75008 Paris (FR)**

(54) **Composition cosmétique de particules monodisperses**

(57)     La présente invention est relative à une composition cosmétique comportant :
- un milieu physiologiquement acceptable, anhydre et non pulvérulent, comportant une phase huileuse,
- des particules monodisperses aptes à former un réseau ordonné de particules monodisperses sur un support sur lequel la composition est appliquée.

EP 1 913 933 A1

## Description

La présente invention concerne les compositions cosmétiques et plus particulièrement mais non exclusivement celles destinées au maquillage des matières kératiniques, notamment la peau, les lèvres, les ongles ou les cils.

## Arrière-plan

Il est connu d'utiliser dans les compositions de maquillage des pigments et colorants.

L'utilisation de tels pigments et colorants peut toutefois soulever des difficultés.

Ainsi, les pigments et colorants peuvent présenter une résistance aux ultraviolets relativement faible et s'altérer à la lumière.

En outre, lorsque la couleur est apportée par un phénomène d'absorption, la coloration produite peut ne pas être aussi vive et lumineuse que souhaitée.

Enfin, le choix des pigments et colorants utilisables en cosmétique peut s'avérer insuffisant.

Les pigments et colorants peuvent encore imposer des contraintes de formulation.

Il est connu pour obtenir un effet goniochromatique d'utiliser des pigments interférentiels. Ceux-ci sont néanmoins de fabrication relativement complexe et coûteuse.

Un effet goniochromatique présent dans la formulation peut encore être apporté par un réseau ordonné de particules monodisperses, comme enseigné notamment dans la demande WO 00/47167. Malgré la relative ancienneté de cette publication, il n'existe pas encore sur le marché à ce jour, à la connaissance de la demanderesse, de produit cosmétique permettant d'obtenir une coloration vive et lumineuse pendant une durée acceptable par le consommateur au moyen d'un réseau ordonné de particules monodisperses après application sur les matières kératiniques.

La publication WO 02/056854 de la société demanderesse divulgue une composition irisée pour application topique, comprenant au moins un tensioactif hydrosoluble et des particules monodisperses en dispersion aqueuse, ces particules ayant une taille moyenne en nombre allant de 50 à 300 nm et la quantité de ces particules étant d'au moins 3 % en poids par rapport au poids total de la composition.

La demande WO 05/018566 divulgue un système topique pour l'application sur la peau, comportant un réseau cristallin colloïdal dans une phase hydrophile et au moins une phase contenant une huile.

La publication WO 2006/097332 divulgue une solution éthanolique anhydre avec une concentration massique de 0,2 % de particules monodisperses de silice pour faire cristalliser les particules à partir des cheveux.

## Résumé

Il existe un besoin pour améliorer encore les compositions permettant de produire une couleur grâce à au moins un réseau ordonné de particules monodisperses, un tel réseau étant encore appelé parfois « cristal photonique ».

Une composition cosmétique selon l'invention peut comporter, selon un aspect de l'invention :

- un milieu physiologiquement acceptable, anhydre et non pulvérulent, comportant une phase huileuse,
- des particules monodisperses aptes à former un réseau ordonné de particules monodisperses sur un support sur lequel la composition est appliquée.

Par « non pulvérulent » il faut comprendre que le milieu comporte au moins une phase liquide ou gélifiée, éventuellement thixotropique. La composition est ainsi différente d'une poudre.

Par « anhydre » il faut comprendre que la teneur massique en eau est inférieure ou égale à 5 %, mieux 2 %, encore mieux 0.5 %. La composition peut notamment ne pas comporter d'eau ajoutée intentionnellement durant la préparation de la composition.

L'invention permet d'obtenir une application et une tenue confortable, y compris sur des régions sensibles telles que les lèvres par exemple, à la différence d'un milieu aqueux qui peut s'avérer difficile à appliquer sur les substrats kératiniques sans obtenir un séchage rapide accompagné d'une sensation d'inconfort et de sècheresse.

La phase huileuse peut être présente en quantité suffisante dans la composition afin d'obtenir une composition liquide ou gélifiée et non pulvérulente.

Dans un exemple de mise en oeuvre de l'invention, le rapport en poids de la phase huileuse sur le poids total de particules (particules monodisperses et éventuellement autres particules) présentes dans la composition est supérieur à 0,5, de préférence à 0,75.

L'arrangement cristallin entre les particules monodisperses peut être obtenu lors de la préparation de la formulation ou lors du séchage des particules sur la peau. Dans le cas où le réseau cristallin n'est pas préformé mais se réalise lors du séchage, les particules sont de préférence des particules chevelues. Dans le cas où l'arrangement est obtenu lors de la préparation de la composition, les méthodes suivantes peuvent par exemple être utilisées :

- réalisation d'un cristal photonique de particules monodisperses puis ajout d'une phase huileuse. Cette phase huileuse peut contenir un polymère et venir gonfler le cristal photonique formé,
- réalisation d'un cristal colloïdal en solution aqueuse en présence d'une phase huileuse puis évaporation progressive de la phase aqueuse,
- réalisation d'un cristal colloïdal en solution huileuse par les techniques classiques (sédimentation, cristallisation par confinement physique, etc...)

De préférence, pour éviter tout phénomène d'agrégation, les particules monodisperses sont de préférence hydrophobes. Des particules de polystyrène ou de PMMA conviennent tout particulièrement ainsi que des particules chevelues portant un coeur monodisperse et un ou plusieurs polymères greffés permettant une bonne dispersion et une bonne stabilisation dans la phase huileuse.

Un polymère pourra également être présent dans la composition afin par exemple d'épaissir ou de gélifier la composition mais également d'améliorer sa tenue.

La composition peut comporter au moins 15 % en poids, mieux 20 % en poids, par rapport au poids total de la composition, de particules monodisperses ayant une taille moyenne de préférence comprise entre 80 nm et 500 nm, mieux entre 150 nm et 450 nm, aptes à former un réseau ordonné de particules monodisperses sur le support sur lequel la composition est appliquée.

Une telle teneur en particules monodisperses, combinée à la sélection ci-dessus en taille, facilite la formation d'un réseau ordonné compact de particules monodisperses présentant des propriétés optiques intéressantes, notamment une réflexion de la lumière dans le domaine du visible ou proche du visible.

La concentration relativement importante de particules peut faciliter la formation d'un réseau cristallin, à l'aide d'un applicateur cosmétique par exemple. Une concentration relativement élevée peut en effet conduire à une préorganisation des particules par répulsion stérique dans la composition et/ou lors du séchage de celle-ci.

Les particules peuvent former un réseau cristallin compact après application. Le réseau peut éventuellement être discontinu avec la présence de fractures et de dislocations.

Une approximation de la longueur d'onde λ de la lumière diffractée par le réseau est donnée par la relation de Bragg :

$$m\lambda = 2nd\sin\theta,$$

avec *m* l'ordre de diffraction, *n* l'indice de réfraction moyen du milieu diffractant, *d* la distance entre deux plans diffractant et θ l'angle de Bragg entre la lumière incidente et le plan diffractant.

La longueur d'onde diffractée est donc principalement dépendante de l'angle d'observation et de la distance entre les particules. Lorsque le réseau formé est compact, cette distance dépend principalement de la taille des particules. Il est donc possible d'obtenir différentes colorations goniochromatiques en faisant varier la taille des particules présentes.

Il est également possible d'obtenir une réflexion dans le domaine UV (protection contre les UV) ou dans le domaine IR (revêtement antichaleur).

Le milieu peut comporter une phase huileuse volatile ou non volatile.

En présence d'une phase volatile, la distance entre les particules variant au cours du séchage, il est possible d'obtenir relativement facilement des compositions cosmétiques ayant une variation continue (du rouge au bleu) de la couleur au cours du séchage après application, ce qui amène un effet ludique pour le consommateur.

L'invention peut également permettre de former un dépôt coloré après application d'une composition initialement incolore.

L'invention peut permettre de réaliser, si on le souhaite, une composition cosmétique dépourvue de colorant ou de pigment, la couleur étant produite par le réseau ordonné de particules monodisperses.

L'invention peut également permettre de réaliser un dépôt coloré sensible à un stimulus extérieur tel que par exemple la température, l'humidité ou le rayonnement ultraviolet.

Un tel stimulus peut exercer une influence sur la distance entre les particules du réseau et ainsi modifier la couleur, comme expliqué précédemment.

La distance entre les particules peut être modifiée en raison par exemple d'une variation de la dimension des particules sous l'effet du stimulus extérieur et/ou d'une variation de la distance entre les particules à taille sensiblement constante, due par exemple à une variation des forces de répulsion entre celles-ci et/ou à une variation de la taille d'au moins un composé présent entre les particules.

L'indice de réfraction du milieu peut éventuellement varier sous l'effet du stimulus extérieur, par exemple la température.

L'invention peut permettre d'obtenir une coloration durable et lumineuse sur une large surface.

**Particules monodisperses**

L'expression « particules monodisperses » désigne selon l'invention des particules dont la taille moyenne présente un coefficient de variation CV inférieur ou égal à 15 %.

Le coefficient de variation CV est défini par la relation $CV = \dfrac{s}{D}$, $s$ étant l'écart-type de la distribution en taille des particules et $D$ la taille moyenne de celles-ci.

La taille moyenne $D$ et l'écart-type $s$ peuvent être mesurés sur 250 particules par analyse d'une image obtenue à l'aide d'un microscope électronique à balayage, par exemple celui de référence S-4 500 de la société HITACHI. Un logiciel d'analyse d'image peut être utilisé pour faciliter cette mesure, par exemple le logiciel Winroof®, commercialisé par la société Mitani Corporation.

De préférence, le coefficient de variation des particules monodisperses est inférieur ou égal à 10 %, mieux inférieur ou égal à 7 %, voire mieux encore inférieur ou égal à 5 %, étant par exemple sensiblement de l'ordre de 3,5 %. Une faible dispersion de la taille des particules peut être favorable à la qualité du réseau cristallin compact formé et donc à l'obtention de couleurs vives et brillantes.

La taille moyenne $D$ des particules monodisperses peut être comprise généralement entre 80 et 500 nm, mieux entre 100 et 500 nm ou 150 et 450 nm, pouvant être choisie en fonction par exemple de la ou des couleurs à obtenir et du milieu environnant.

Une plage préférentielle de taille moyenne va de 150 à 450 nm, mieux de 190 à 310 nm, pour l'obtention de couleurs dans le domaine visible. La taille moyenne peut aller de 80 à 200 nm pour la filtration des UV.

Selon un aspect de l'invention, la teneur massique en particules monodisperses dans les compositions selon l'invention peut être d'au moins 15 % en poids, par rapport au poids total de la composition. Elle peut par exemple aller de 15 à 70 % en poids, étant par exemple supérieure ou égale à 20 % en poids, notamment strictement supérieure à 20 % en poids, supérieure ou égale à 25 %, 30 %, 35 %, 40 % ou 45 % en poids, par rapport au poids total de la composition. Une teneur autre, allant par exemple de 1 à 70 % en poids peut être admise selon certains autres aspects de l'invention. Lorsque les particules monodisperses sont contenues dans une phase huileuse, la teneur massique en particules monodisperses dans cette phase huileuse peut être comprise entre 0,00 1% et 70 % en poids, relativement au poids de la phase huileuse.

Suivant la concentration en particules utilisées dans la composition, le réseau périodique formé peut être monocouche ou multicouche, compact ou non.

La forme des particules monodisperses doit être compatible avec la formation d'un réseau ordonné de particules monodisperses.

Le réseau formé peut être au moins partiellement cubique centré, cubique face centrée ou hexagonal compact ou hybride, formé à partir de ces agencements, ou autres.

Différents exemples de formation d'un réseau cristallin à partir de particules monodisperses sont donnés dans la publication Xia et al., Adv. Mater., 12, 693-713 (2000).

De préférence, la forme des particules monodisperses est sphérique, mais d'autres formes, présentant une symétrie axiale notamment, sont possibles.

Les particules monodisperses peuvent être monomatières ou composites.

Les particules monodisperses peuvent être pleines ou creuses. Des particules monodisperses creuses présentent une densité moindre que des particules pleines et permettent donc d'occuper plus de volume pour une même concentration massique. Dans le cas où les particules monodisperses sont constituées d'un matériau de forte densité, par exemple un matériau inorganique, les particules creuses permettent de limiter les phénomènes de sédimentation dans la composition.

La présence d'air ou d'un autre gaz à l'intérieur des particules après séchage permet d'obtenir une grande différence d'indice de réfraction entre les particules et le milieu environnant, ce qui est favorable en terme d'intensité du pic de diffraction et donc du développement d'une coloration très intense. On peut rajouter de nombreux composés non volatils dans la composition ou sur la composition sans risque de perdre la couleur et de se retrouver avec une composition transparente.

Les particules monodisperses peuvent être poreuses ou non. La présence de pores de petite taille au sein des particules peut diminuer l'indice de réfraction de ces particules.

L'indice de réfraction $n_p$ des particules monodisperses est différent de celui $n_c$ du milieu continu s'étendant autour des particules après application de la formulation et la différence de ces indices de réfraction est de préférence supérieure ou égale à 0,02, mieux supérieure ou égale à 0,05, encore mieux supérieure ou égale à 0,1, étant par exemple comprise entre 0,02 et 2, notamment entre 0,05 et 1.

Une différence d'indice de réfraction $n_p$-$n_c$ trop faible peut nécessiter un grand nombre de couches de particules du réseau ordonné pour l'obtention du résultat recherché. Une différence d'indice trop importante peut accentuer les phénomènes de diffusion de la lumière par la couche et amener un blanchiment du dépôt après application.

L'indice de réfraction des particules monodisperses est défini comme étant l'indice de réfraction moyen. Dans le cas de particules composites, il est calculé de façon linéaire en fonction de la proportion volumique de chaque composant.

L'indice de réfraction des particules monodisperses peut être supérieur ou égal à celui du milieu, étant par exemple supérieur ou égal à 1,4, notamment compris entre 1,4 et 1,7.

Toutes les particules monodisperses correspondant à une même taille moyenne D peuvent avoir sensiblement le même indice de réfraction.

Les particules monodisperses peuvent être colorées, c'est-à-dire non blanches, par exemple pour renforcer l'intensité de la couleur produite et/ou éviter un phénomène de blanchiment de la composition après application sur les matières kératiniques.

Un exemple de particule colorée utilisée pour former un cristal colloïdal est donné dans la publication WO 05/012961.

La couleur des particules monodisperses peut être apportée par le choix du ou des matériaux constituant chaque particule monodisperse. Elle peut avoir pour effet d'augmenter l'absorption de la lumière par les particules et de diminuer la diffusion.

Les particules monodisperses peuvent notamment incorporer au moins un pigment ou colorant, organique ou inorganique, celui-ci pouvant le cas échéant être fluorescent et présenter une fluorescence dans l'ultraviolet ou l'infrarouge.

Les particules monodisperses peuvent comporter un composé inorganique, voire être entièrement minérales.

Lorsque les particules monodisperses sont inorganiques, celles-ci peuvent par exemple comporter au moins un oxyde, notamment métallique et choisi par exemple parmi les oxydes de silice, de fer, de titane, d'aluminium, de chrome, de zinc, de cuivre, de zirconium et de cérium et leurs mélanges. Les particules monodisperses peuvent également comporter un métal, notamment du titane, de l'argent, de l'or, de l'aluminium, du zinc, du fer, du cuivre, et leurs mélanges et alliages.

Les particules monodisperses peuvent comporter un composé organique, voire être entièrement organiques.

Parmi les matériaux pouvant convenir pour réaliser des particules monodisperses organiques, on peut citer les polymères, notamment à chaîne carbonée ou siliconée, par exemple le polystyrène (PS), le polymétacrylate de méthyle (PMMA), les polymères de silicone.

Lorsque les particules monodisperses sont composites, celles-ci peuvent par exemple comporter un coeur et une écorce réalisés dans des matières différentes, par exemple des matières organiques et/ou minérales.

Lorsque les particules monodisperses sont composites, le matériau du coeur ou de l'écorce par exemple peut être choisi par exemple afin d'améliorer la stabilité dans le milieu des particules monodisperses, d'augmenter leur indice de réfraction et/ou pour colorer celles-ci et/ou pour leur conférer une fluorescence ou une susceptibilité magnétique.

Le coeur peut être constitué d'un matériau insoluble dans le milieu contenant les particules, par exemple un matériau inorganique, comme la silice par exemple, ou un matériau organique, comme un polymère acrylique par exemple.

L'écorce peut être constituée de chaînes polymériques, lesquelles peuvent être solubles dans le milieu contenant les particules, les chaînes polymériques pouvant comporter des polymères greffés à la surface du coeur des particules monodisperses, qui lui peut être insoluble dans le milieu.

De telles particules à coeur et chaînes polymériques, encore appelées particules « chevelues », peuvent être stabilisées dans le milieu non seulement par des interactions électrostatiques mais également par des interactions stériques de type volume exclu.

La stabilisation supplémentaire et le volume apporté par les chaînes polymériques permettent d'incorporer facilement d'autres composants dans la composition sans risque de déstabilisation et d'agrégation des particules. Ces autres composants sont par exemple des agents de coloration ou des charges destinées par exemple à modifier l'aspect de la composition ou du support revêtu de celle-ci.

Les chaînes polymériques peuvent comporter des chaînes de polymères greffées, lesquelles peuvent contenir des fonctions chimiques (acide carboxylique, amine, amide, thiol, etc...) susceptibles d'interagir avec les matières kératiniques et d'améliorer l'adhésion de la composition sur le support recouvert.

Les chaînes polymériques peuvent également améliorer la tenue du réseau de particules après application sur les matières kératiniques.

Des exemples de particules « chevelues » sont donnés par exemple dans la publication Ishizu et al., Kagaku To Kogyo, 57(7) (2004) dans le cas d'un coeur de polymère ou dans la publication Okubo et al., Colloid & Polymer Science, 280(3), pp290-295 (2002) dans le cas d'un coeur de silice et de polymères poly- méthacrylate de méthyle ou poly(styrène co anhydride maléique) en écorce.

Le cas échéant, la présence d'une écorce peut permettre d'encapsuler dans celle-ci un composé pour lequel un contact direct avec les matières kératiniques ou le milieu n'est pas souhaitable.

Les particules monodisperses composites peuvent encore comporter des inclusions d'un premier matériau dans une matrice d'un second matériau. Par exemple, le premier matériau peut présenter un indice de réfraction élevé permettant d'accroître l'indice de réfraction global de la particule. La particule peut par exemple comporter des inclusions de nanoparticules, par exemple des nanoparticules d'oxyde de titane.

Les particules monodisperses peuvent être fabriquées selon des procédés de synthèse tels que décrits par exemple dans la publication Xia et al, Adv. Mater., 12, 693-713 (2000), incorporé par référence.

Comme références commerciales de particules monodisperses pouvant convenir, on peut citer Optibind® (polystyrène) 216 ou 290 nm microparticles de la société Seradyn, Eposter® MX-100W (PMMA) et Eposter® MX-200W (PMMA) de la société Nippon Shokubai.

Des exemples de particules monodisperses à base de silice et de particules magnétiques sont décrits dans l'article XU et al., Chem. Mater. 14, 1249-1256 (2002).

**Milieu contenant les particules monodisperses**

Selon l'invention, les particules monodisperses peuvent être contenues au moins avant l'application dans un milieu physiologiquement acceptable permettant la formation sur le support sur lequel la composition est appliquée d'un réseau ordonné de particules monodisperses.

Par « milieu physiologiquement acceptable », synonyme de l'expression « milieu cosmétiquement acceptable », on désigne un milieu non toxique et susceptible d'être appliqué sur les matières kératiniques d'êtres humains, notamment la peau, les muqueuses ou les phanères.

Le milieu physiologiquement acceptable est généralement adapté à la nature du support sur lequel doit être appliquée la composition ainsi qu'à la forme sous laquelle la composition est destinée à être conditionnée.

Les particules monodisperses peuvent être contenues dans une phase liquide, notamment huileuse, ou gélifiée.

Le milieu contenant les particules monodisperses peut être entièrement liquide ou contenir d'autres particules, le cas échéant. Le rapport en poids de la phase huileuse sur le poids total de particules peut être supérieur à 0,5, mieux 0,75.

Le milieu peut être choisi de manière à favoriser la dispersion des particules dans le milieu avant l'application de celui-ci, afin d'éviter une agrégation des particules.

Le milieu peut être choisi de telle sorte que le réseau ordonné de particules monodisperses se forme par empilement régulier de celles-ci, après l'application sur les matières kératiniques, le réseau n'existant pas dans la composition avant l'application et se formant par exemple lors de l'évaporation d'un solvant contenu dans la composition.

L'indice de réfraction du milieu présente avantageusement, comme indiqué précédemment, une différence avec celui des particules monodisperses, cette différence étant en valeur absolue de préférence supérieure ou égale à 0,02, mieux supérieure ou égale à 0,05, notamment entre 0,05 et 1, mieux encore supérieure ou égale à 0,1.

Le milieu peut être monophasique ou multiphasique et comporter ou non des solides autres que les particules monodisperses, notamment des plus petites particules ou des plus grosses particules.

De préférence, en présence d'autres corps solides que les particules monodisperses, la quantité de ces corps sera suffisamment faible pour ne pas gêner la formation du réseau ordonné de particules monodisperses et l'obtention du résultat souhaité en termes de coloration notamment.

Caractère volatil

Le milieu dans lequel se forme le réseau ordonné de particules monodisperses peut s'évaporer ou non après l'application de la composition.

De préférence, le milieu peut comporter un solvant volatil. Par «solvant volatil», on entend au sens de l'invention tout liquide susceptible de s'évaporer au contact de la peau, à température ambiante et sous pression atmosphérique.

Le milieu peut notamment être choisi de manière à ce que la composition contienne au moins 10 %, voire au moins 30 % de solvant volatil.

Phase huileuse (encore appelée phase grasse)

La composition selon l'invention peut comporter dans certains exemples de mise en oeuvre une phase huileuse. Les particules monodisperses peuvent être contenues ou non dans cette phase huileuse.

La phase huileuse peut notamment être volatile.

L'introduction d'une ou plusieurs huiles pourra se faire de façon à ne pas perdre l'effet de coloration ou la réflectance spectrale recherché.

La composition peut comporter une huile telle que par exemple les esters et les éthers de synthèse, les hydrocarbures linéaires ou ramifiés, d'origine minérale ou synthétique, les alcools gras ayant de 8 à 26 atomes de carbone, les huiles fluorées partiellement hydrocarbonées et/ou siliconées, les huiles de silicone comme les polyméthylsiloxanes (PDMS) volatiles ou non à chaîne siliconée linéaire ou cyclique, liquides ou pâteux à température ambiante et leurs mélanges, d'autres exemples étant donnés ci-après.

Une composition conforme à l'invention, et par exemple le milieu physiologiquement acceptable, peut comprendre au moins une huile volatile.

Huiles volatiles

Au sens de la présente invention, on entend par "huile volatile", une huile (ou milieu non aqueux) susceptible de s'évaporer au contact de la peau en moins d'une heure, à température ambiante et à pression atmosphérique.

L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées d'origine animale ou végétale ayant de 8 à 16 atomes de carbone, et notamment les alcanes ramifiés en $C_8$-$C_{16}$ (appelées aussi isopa-raffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité $\leq$ 8 centistokes (8 x $10^{-6}$ m$^2$/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluoro-rométhylcyclopentane, et leurs mélanges.

Il est également possible d'utiliser un mélange des huiles précédemment citées.

Huiles non volatiles

Une composition selon l'invention peut comporter une huile non volatile.

Au sens de la présente invention, on entend par "huile non-volatile", une huile ayant une pression de vapeur inférieure à 0,13 Pa et notamment des huiles de masse molaire élevée.

Les huiles non volatiles peuvent notamment être choisies parmi les huiles hydrocarbonées le cas échéant fluorées et/ou les huiles siliconées non volatiles.

Comme huile hydrocarbonée non volatile pouvant convenir à la mise en oeuvre de l'invention, on peut notamment citer :

-    les huiles hydrocarbonées d'origine animale,
-    les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL,
-    les huiles hydrocarbonées d'origine minérale ou synthétique comme par exemple :

    •    les éthers de synthèse ayant de 10 à 40 atomes de carbone ;
    •    les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,

- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$.

Les esters peuvent être notamment choisis parmi les esters, notamment d'acide gras comme par exemple :

- l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C12 à C15, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyols, et les esters de pentaétrythritol, comme le tétrahydroxystéarate/tétraisostéarate de dipentaérythritol,
- les esters de dimères diols et dimères diacides tels que les Lusplan DD-DA5® et Lusplan DD-DA7®, commercialisés par la société NIPPON FINE CHEMICAL et décrits dans la demande FR 03 02809,
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination Cetiol CC®, par Cognis,
- les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,

- et leurs mélanges.

Le milieu peut comporter au moins un composé présentant une liaison OH, notamment une fonction alcool, en une teneur massique par exemple supérieure ou égale à 5 %, mieux 10 %. Un tel composé peut augmenter l'évaporation sans perturber la formation d'un réseau ordonné.

Le milieu peut comporter un alcool, comme l'éthanol ou l'isopropanol, par exemple, ou un dérivé du glycol, notamment l'éthylène glycol ou le propylène glycol.

Le milieu peut être transparent ou translucide, et coloré ou non. Le milieu contenant les particules monodisperses peut ne contenir aucun pigment ou colorant. La coloration du milieu peut correspondre à l'ajout d'un agent de coloration additionnel.

La couleur du milieu correspond par exemple à l'une des couleurs susceptibles d'être générées par le réseau ordonné de particules monodisperses, par exemple la couleur produite par le réseau lorsqu'observé sous incidence normale.

La couleur du milieu peut également être noire afin de limiter la diffusion de la lumière.

Agents de coloration

Le réseau ordonné de particules monodisperses peut permettre d'obtenir assez facilement les couleurs vert, rouge ou bleu. Le domaine coloriel peut être étendu grâce à la présence d'un agent de coloration additionnel, par exemple un colorant, un pigment absorbant ou un pigment à effet, par exemple à une concentration allant de 0,1 à 15 % en masse.

Par « *pigment à effet* » on entend, entre autres, des particules réfléchissantes, des nacres, des agents de coloration goniochromatique ou des pigments diffractants, tels que définis ci-dessous.

La présence de pigments d'une taille relativement grande, tels que des nacres par exemple, peut ne pas empêcher la formation du réseau à côté des particules de pigment, mais au contraire favoriser sa formation en améliorant le confinement des particules monodisperses, les grosses particules pouvant s'introduire dans certaines dislocations du réseau.

Le milieu peut ainsi comporter des particules plus grosses ayant une taille au moins 3, mieux 5 fois supérieure à celle des particules monodisperses, encore mieux 10 fois plus supérieure. Ces grosses particules peuvent être des particules d'un pigment ou d'une charge non colorante. Le milieu peut ainsi comporter au moins un pigment à effet.

La présence de particules monodisperses permet de réaliser un réseau périodique après application sur les matières kératiniques. Ce réseau permet d'obtenir un effet coloriel par diffraction de la lumière et la demanderesse a trouvé qu'il est possible d'associer un deuxième effet optique grâce à un pigment à effet tout en conservant le réseau périodique. Les deux effets optiques seront additionnels et la présence du pigment permet donc d'étendre le domaine coloriel et les effets optiques obtenus par le réseau formé sur les matières kératiniques.

Le pigment à effet peut être présent dans la formulation à une concentration comprise entre 0,1 et 70 %, de préférence 1 à 50 %, de préférence 5 à 20 %.

Particules réfléchissantes

Les particules réfléchissantes peuvent permettre de créer des points de surbrillance visibles à l'oeil nu.

Les particules réfléchissantes peuvent présenter des formes variées. Ces particules peuvent être notamment en forme de plaquettes ou globulaires, en particulier sphériques. Ces particules peuvent comporter un substrat recouvert d'un matériau réfléchissant.

Le substrat peut être choisi parmi les verres, les oxydes métalliques, les alumines, les silices, les silicates, notamment les aluminosilicates et les borosilicates, le mica, le mica synthétique, les polymères synthétiques et leurs mélanges.

Le matériau réfléchissant peut comporter une couche de métal ou d'un composé métallique.

Des particules à substrat de verre revêtu d'argent, en forme de plaquettes, sont vendues sous la dénomination METASHINE par la société Nippon Sheet Glass.

A titre d'exemple de particules réfléchissantes, on peut encore citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Conviennent également à l'invention, les pigments de la gamme METASHINE 1080R commercialisée par la société NIPPON SHEET GLASS CO. LTD. Ces pigments, plus particulièrement décrits dans la demande de brevet JP 2001-11340, sont des paillettes de verre C-GLASS comprenant 65 à 72 % de $SiO_2$, recouvertes d'une couche d'oxyde de titane de type rutile ($TiO_2$). Ces paillettes de verre ont une épaisseur moyenne de 1 micron et une taille moyenne de 80 microns, soit un rapport en taille moyenne/épaisseur moyenne de 80. Elles présentent des reflets bleus, verts, jaunes ou de teinte argent selon l'épaisseur de la couche de $TiO_2$.

On peut encore citer les particules de dimension comprise entre 80 et 100 $\mu$m, comportant un substrat de mica synthétique (fluorophlogopite) revêtu de dioxyde de titane représentant 12 % du poids total de la particule, vendues sous la dénomination PROMINENCE par la société NIHON KOKEN.

Les particules réfléchissantes peuvent encore être choisies parmi les particules formées d'un empilement d'au moins deux couches à indices de réfraction différents. Ces couches peuvent être de nature polymérique ou métallique et notamment inclure au moins une couche polymérique. Ainsi, les particules réfléchissantes peuvent être des particules dérivant d'un film polymérique multicouche. De telles particules sont notamment décrites dans WO 99/36477, US 6 299 979 et US 6 387 498. Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.

Nacres

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres Flamenco commercialisées par la société ENGELHARD et les nacres TIMIRON commercialisées par la société MERCK.

Agents de coloration goniochromatiques

Les agents de coloration goniochromatiques au sens de la présente invention présentent un changement de couleur, encore appelé « color flop », en fonction de l'angle d'observation, supérieur à celui que l'on peut rencontrer avec des nacres.

L'agent de coloration goniochromatique peut être choisi par exemple parmi les structures multicouches interférentielles et les agents de coloration à cristaux liquides.

Des exemples de pigments multicouches interférentiels symétriques utilisables dans des compositions réalisées conformément à l'invention sont par exemple : CHROMAFLAIR par la société FLEX ; SICOPEARL par la société BASF ; pigments XIRONA par la société MERCK (Darmstadt), les pigments INFINITE COLORS de la société SHISEIDO et les pigments COLOR RELIEF de la société CCIC.

On peut encore utiliser des agents de coloration goniochromatiques à structure multicouche comprenant une alternance de couches polymériques par exemple du type naphtalate de polyéthylène et téréphtalate de polyéthylène. De tels agents sont notamment décrits dans WO-A-96/19347 et WO-A-99/36478.

On peut citer, à titre d'exemple de pigments à structure multicouche polymérique, ceux commercialisés par la société 3M sous la dénomination COLOR GLITTER ou ceux commercialisés par la société Venture Chemical sous la dénomination Micro Glitter Pearl.

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celle commercialisées sous la dénomination HELICONE® HC par la société SICPA.

La composition peut en outre comporter des fibres goniochromatiques dispersées. De telles fibres peuvent par exemple présenter une taille comprise entre 50 $\mu$m et 2 mm. Des fibres goniochromatiques à structure bicouche polyéthylène téréphtalate/nylon-6 sont commercialisées par la société TEIJIN sous la dénomination MORPHOTEX et MORPHOTONE.

Pigments diffractants

Par pigment diffractant on entend un pigment comportant un motif périodique constituant un réseau de diffraction. La distance entre les motifs périodiques étant du même ordre de grandeur que la lumière visible ce pigment pourra diffracter la lumière et produire par exemple un effet arc-en ciel.

De tels pigments sont disponibles commercialement sous la dénomination SPECTRAFLAIR auprès de la société JDS Uniphase Corporation.

De tels pigments peuvent également être réalisés suivant les méthodes enseignées par les brevets US6818051, US6894086 et EP1634619. Ces brevets décrivent des pigments constitués d'un réseau en 3 dimensions de particules de silice similaire à la structure des opales. Des structures opales inverses peuvent également être obtenues et utilisées.

Taille des particules autres que monodisperses

Le milieu peut comporter des particules plus petites ayant une taille moyenne D inférieure à celle des particules monodisperses, d'un facteur d'au moins 2, mieux d'au moins 3, afin de permettre leur insertion dans les vides laissés entre les particules monodisperses du réseau.

Ces particules interstitielles peuvent être minérales ou organiques et peuvent améliorer la cohésion du réseau ou modifier l'absorption de la lumière par les couches du réseau.

Comme exemple de particules interstitielles, on peut citer les nanoparticules de dioxyde de titane, de silice, d'oxyde de fer, de noir de carbone, de taille moyenne allant de 5 à 150 nm, par exemple de 10 à 100 nm.

Comme autre exemple de particules interstitielles, on peut mentionner des particules d'un polymère, lequel est par exemple à l'état déjà polymérisé dans la composition avant son application sur les matières kératiniques, le milieu comportant par exemple un latex.

La taille des particules interstitielles peut, le cas échéant, varier en fonction d'un stimulus extérieur et/ou de la concentration d'un composé dans le milieu. Les particules interstitielles peuvent être hydroabsorbantes. La taille des particules peut alors par exemple varier en fonction de la concentration en eau dans le milieu.

La variation de taille des particules interstitielles peut, le cas échéant, exercer une action sur la distance entre les particules monodisperses et ainsi avoir une action sur la couleur produite par le réseau.

Présence d'un polymère

Le milieu peut comporter au moins un polymère permettant d'améliorer la tenue du réseau après sa formation.

Ce polymère est par exemple à l'état non entièrement polymérisé et/ou réticulé dans la composition avant l'application de celle-ci et son séchage.

Lorsque le milieu contient un polymère qui n'est pas entièrement polymérisé et/ou réticulé avant l'application de la composition sur les matières kératiniques, la réticulation et/ou polymérisation peut avoir lieu après l'application de la composition sur les matières kératiniques.

La polymérisation et/ou réticulation peut intervenir par exemple après la formation du réseau de particules monodisperses ou en variante avant celle-ci et/ou concomitamment à celle-ci.

Le milieu peut comporter un polymère filmogène.

Polymère filmogène

Dans la présente invention, on entend par « polymère filmogène », un polymère apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconée.

La composition peut comporter une phase huileuse et le polymère filmogène peut être présent dans cette phase huileuse. Le polymère peut être un polymère en dispersion ou un polymère amphiphile ou associatif.

Par « *polymère en dispersion* » on entend des polymères non solubles dans la phase huileuse et présents sous forme de particules de taille variable. Le polymère peut être réticulé ou non. La taille de particules moyenne est typiquement comprise entre 25 et 500 nm, de préférence entre 50 et 200 nm. Les polymères en dispersion huileuse suivants peuvent être utilisés : polymères acryliques ou méthacryliques, polymères siliconés, polymères acrylique siliconés.

Par « *polymères amphiphiles ou associatifs* » on entend des polymères comportant une ou plusieurs parties hydrophobes par lesquelles les polymères s'associent ou interagissent. Les polymères amphiphiles comme les polymères à blocs à base de polystyrène peuvent notamment être utilisés.

La composition peut comporter une phase huileuse et le polymère filmogène peut être présent dans cette phase huileuse. Le polymère pourra alors être en dispersion ou en solution. Les polymères de type NAD (non aqueous dispersion) ou des microgels (par exemple les KSG) peuvent être utilisés, ainsi que les polymères du type PS-PA ou les copolymères à base de styrène (Kraton, Regalite).

Comme exemples de dispersions non aqueuses de polymère filmogène lipodispersibles sous forme de dispersions non aqueuses de particules de polymère dans une ou plusieurs huiles de silicone et/ou hydrocarbonées et pouvant être stabilisées en leur surface par au moins un agent stabilisant, notamment un polymère séquencé, greffé ou statistique, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrites notamment dans le document WO 04/055081.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C30, de préférence en C1-C20, des (méth)acrylates d'aryle, en particulier d'aryle en C6-C10, des (méth)acrylates d'hydroxyalkyle, en particulier d'hydroxyalkyle en C2-C6 .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbomane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Selon un exemple de composition selon l'invention, le polymère filmogène peut être un polymère solubilisé dans une phase grasse liquide comprenant des huiles ou solvants organiques (on dit alors que le polymère filmogène est un polymère liposoluble). De préférence, la phase grasse liquide comprend une huile volatile, éventuellement en mélange avec une huile non volatile.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme exemple de polymères filmogènes liposolubles, on peut citer les copolymères d'ester vinylique et au moins un autre monomère qui peut être un ester vinylique, notamment le néodécanoate de vinyle, le benzoate de vinyle et le t-butyl benzoate de vinyle, une α-oléfine, un alkylvinyléther, ou un ester allylique ou méthallylique.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2 232 303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polyméthylsilsesquioxanes commercialement disponibles, on peut citer celles qui sont commercialisés :

- par la société Wacker sous la référence Resin MK tels que la Belsil PMS MK:
- par la société SHIN-ETSU sous les références KR-220L.

Comme résines siloxysilicates, on peut citer les résines triméthylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'une résine de silicone, telle que celles décrites plus haut, et d'un diorganosiloxane tels que décrits dans le document WO 2004/073626.

Selon un exemple de mise en oeuvre de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences du polymère séquencé sont incompatibles l'une avec l'autre.

De tels polymères sont décrits par exemple dans les documents EP 1 411 069 ou WO 04/028488.

Le polymère filmogène peut être choisi parmi les polymères et/ou copolymères blocs ou statiques comportant notamment les polyuréthanes, polyacryliques, les silicones, les polymères fluorés, les gommes butyliques, les copolymères d'éthylènes, gommes naturelles et les alcools polyvinyliques et leurs mélanges. Les monomères des copolymères blocs ou statiques comprenant au moins une association de monomères dont le polymère résulte à une température de transition vitreuse inférieure à la température ambiante (25 °C) peuvent être choisis parmi notamment le butadiène, l'éthylène, le propylène, l'acrylique, le méthacrylique, l'isoprène, l'isobutène, une silicone et leurs mélanges.

Le polymère filmogène peut être également présent dans la composition sous la forme de particules en dispersion dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Bien entendu, cette liste de polymères n'est pas exhaustive.

De préférence, lorsque le milieu contenant les particules monodisperses contient un polymère filmogène, celui-ci est par exemple une dispersion aqueuse de polymère acrylique, vinylique, fluoré, siliconé ou leurs mélanges.

La teneur massique en polymère(s) filmogène(s) dans la composition contenant les particules monodisperses va par exemple de 0,1 à 10 %.

Lorsque la composition contenant les particules monodisperses contient un polymère non entièrement polymérisé et/ou réticulé, la polymérisation et/ou réticulation peut s'effectuer par amorçage thermique ou par rayonnement ultraviolet.

La polymérisation peut également s'effectuer par ajout d'un initiateur et éventuellement d'un agent réticulant.

Lorsque l'on souhaite réaliser un réseau de particules monodisperses dans le milieu, il est possible d'ajouter un monomère et un initiateur et éventuellement un agent réticulant, puis d'effectuer la polymérisation.

Celle-ci peut avoir lieu au moment de la fabrication de la formulation ou encore après application sur la peau. Cette méthode permet la réalisation de polymères de grande masse moléculaire ou de polymères réticulés. Cela peut permettre de faire varier à façon la rhéologie du système formé.

Le milieu peut également comporter un polymère permettant la formation d'un gel, par exemple avant ou après l'application de la composition sur le support à maquiller.

Polymères permettant la formation d'un gel

La formation d'un gel peut par exemple améliorer la cohésion du réseau de particules monodisperses et/ou rendre celui-ci sensible à un stimulus extérieur et/ou à la concentration d'un composé dans le milieu.

La quantité massique de polymère destiné à la formation d'un gel dans la composition peut être comprise entre 0,5 et 60 %, mieux entre 5 et 40 %.

Le polymère destiné à la formation d'un gel peut polymériser après l'application de la composition sur le support à maquiller. En variante, le gel est formé avant l'application de la composition sur les matières kératiniques, puis appliqué sur celles-ci.

La réalisation du gel peut aussi avoir lieu avant la fabrication de la composition. Il est possible par exemple de réaliser un gel huileux à base d'élastomère de polydiméthylsiloxane à partir d'un réseau de sphères de polystyrène comme cela est décrit dans l'article de H. Fudouzi et al, Langmuir, 19, 9653-9660 (2003).

Un gel convenable peut également être obtenu en mettant à profit la faculté de certains composés, notamment siliconés, à interagir lorsqu'ils sont mis en présence et à constituer à l'issue de leur interaction un réseau polymérique.

Les polymères obtenus par réaction d'un composé X et d'un composé Y, l'un au moins étant siliconé, tels que détaillés ci-après, permettent en effet la formation *in situ*, à pression atmosphérique et à température ambiante, de gels avantageusement biocompatibles. De tels systèmes sont notamment décrits en partie dans le document WO 01/96450 de Dow Corning.

Ainsi, selon un mode de réalisation de l'invention, le milieu peut dériver de la réaction d'hydrosilylation, de condensation ou de réticulation en présence d'un peroxyde d'au moins un composé X et d'au moins un composé Y, l'un au moins des composés X et Y étant siliconé.

Selon une première alternative, une composition conforme à la présente invention peut être obtenue après formation d'un mélange d'au moins un composé X et d'au moins un composé Y, l'un au moins desdits composés X et Y étant siliconé, et lesdits composés X et Y étant susceptibles de réagir ensemble par une réaction d'hydrosilylation, de condensation ou de réticulation en présence d'un peroxyde, lorsqu'ils sont mis en contact l'un avec l'autre, le produit de réaction ainsi obtenu étant ensuite mis en présence des particules monodisperses considérées selon l'invention. Cette alternative est notamment illustrée par l'exemple 1 ci-après.

Selon une seconde alternative, une composition conforme à la présente invention peut être obtenue à l'issue du mélange extemporané, voir directement sur le support d'application considéré, d'une première composition contenant au moins un composé X et d'une deuxième composition contenant au moins un composé Y, les composés X et Y étant tels que définis ci-dessus, avec au moins l'une des première et deuxième compositions contenant en outre des particules monodisperses telles que considérées selon l'invention.

Les composés X et Y porteurs de groupes polaires sont avantageusement susceptibles de réagir ensemble sur la peau à température ambiante (20 ± 5°C) et pression atmosphérique, seuls ou en présence d'un catalyseur, par une réaction d'hydrosilylation, de condensation ou de réticulation en présence d'un peroxyde.

Par composé siliconé, on entend un composé dont la chaîne principale est formée majoritairement d'unités organosiloxanes.

De préférence, ces composés X et Y sont des polymères. De préférence encore, le composé X et le composé Y sont siliconés.

Selon un mode de réalisation, ils sont choisis parmi les silicones, de préférence élastomères réactives dites "room temperature vulcanization" à propriétés adhésives.

Parmi les composés siliconés cités ci-après, certains peuvent présenter en outre à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Selon un mode de réalisation particulier, l'un au moins des composés X et Y est porteur d'au moins un groupe polaire notamment susceptible de former au moins une liaison hydrogène avec la peau.

Par groupe polaire, on entend un groupe comportant des atomes de carbone et d'hydrogène dans sa structure chimique et au moins un hétéroatome (tel que O, N, S et P), tel que ledit groupe est apte à établir au moins une liaison hydrogène avec la peau.

Selon un premier mode de réalisation, les composés siliconés sont susceptibles de réagir par hydrosilylation, cette réaction pouvant être de manière simplifiée schématisée comme suit :

$$\text{-Si-H} + CH_2\text{=HC-X} \rightarrow \text{-Si-}CH_2\text{-}CH_2\text{-X}$$

Dans ce cas, le composé X peut être choisi parmi les composés siliconés comprenant une chaîne principale siliconée et au moins deux groupements aliphatiques insaturés, qui sont pendants à la chaîne principale (groupe latéral) ou situés aux extrémités de la chaîne principale du composé (groupe terminal).

A titre d'exemple d'une combinaison de composés X et Y réagissant par hydrosilylation, on peut citer les références suivantes proposée par la société Dow Corning : DC 7-9800 Soft Skin Adhesive Parts A & B, ainsi que la combinaison des mélanges A et B suivants préparés par Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

Selon un autre mode de réalisation, les composés siliconés sont susceptibles de réagir par condensation, soit en présence d'eau (hydrolyse) par réaction de 2 composés porteurs de groupements alcoxysilanes.

Selon une variante, les composés siliconés sont susceptibles de réagir par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Dans ce cas, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

A titre d'exemple d'une combinaison de composés X et Y porteurs de groupements alcoxysilanes et réagissant par condensation, on peut citer la combinaison des mélanges A' et B' suivants préparés par la société Dow Corning :

Mélange A' :

| Ingrédient (Nom INCI) | N°CAS | Teneurs (%) | Fonction |
|---|---|---|---|
| Bis-Trimethoxysiloxyethyl Tetramethyldisilo xyethyl | PMN87176 | 25-45 | Polymère |
| Dimethicone (1) | | | |
| Silica Silylate | 68909-20-6 | 5-20 | Charge |
| Disiloxane | 107-46-0 | 30-70 | Solvant |

Mélange B' :

| Ingrédient (Nom INCI) | N°CAS | Teneurs (%) | Fonction |
|---|---|---|---|
| Disiloxane | 107-46-0 | 80-99 | Solvant |
| Tetra T Butyl Titanate | - | 1-20 | Catalyseur |

Il est à noter que les composés X et Y, identiques, sont réunis dans le mélange A' (cf. (1))

Selon un troisième mode de réalisation, les composés siliconés X et Y sont susceptibles de réagir par réticulation en présence de peroxyde.

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux -CH$_3$ et/ou au moins deux chaînes latérales portant un groupement -CH$_3$.

Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux -CH$_3$ reliés à l'atome de silicium et/ou au moins deux chaînes latérales portant un groupement -CH$_3$.

Quelque soit le mode de réalisation, la réaction d'hydrosilylation, de condensation ou de réticulation en présence d'un peroxyde entre les composés X et Y peut être accélérée par un apport de chaleur en élevant par exemple la température du système entre 25 °C et 180 °C. Le système réagira notamment sur la peau.

Le ratio entre les composés X et Y peut être varié de manière à moduler la vitesse de réaction et donc la vitesse de formation du gel ou encore de manière à adapter les propriétés du gel formé (par exemple sa viscosité ou ses propriétés adhésives) selon les effets recherchés.

En particulier, les composés X et Y peuvent être mis en présence dans un ratio X/Y molaire allant de 0,05 à 20 et mieux de 0,1 à 10.

## Kits

L'invention a encore pour objet des kits comportant une composition selon l'invention.

Ces kits peuvent comporter au moins une composition destinée à former une couche de base, encore appelée « base coat » et/ou une couche de recouvrement, encore appelée « top coat ».

Le kit peut ainsi comporter :

- une première composition à appliquer sur les matières kératiniques,
- une deuxième composition à appliquer sur la première, cette deuxième composition comportant :

  - un milieu physiologiquement acceptable, anhydre et non pulvérulent,
  - des particules monodisperses aptes à former un réseau ordonné de particules monodisperses sur un support sur lequel la composition est appliquée.

Selon une variante de réalisation, l'une des première et deuxième compositions peut comprendre en outre au moins un composé X, et l'autre des première et deuxième compositions peut comprendre en outre au moins un composé Y, les composés X et Y étant tels que définis précédemment.

Selon un autre mode de réalisation, le kit peut comporter :

- une première composition comportant :

  - des particules monodisperses,
  - un milieu anhydre non pulvérulent permettant la formation sur un support sur lequel la composition est appliquée d'un réseau ordonné de particules monodisperses,

- une deuxième composition destinée à la formation d'une couche de base ou de recouvrement.

La couche de base et la couche de recouvrement peuvent être simultanément présentes, le kit pouvant alors comporter :

- une première composition cosmétique comportant :

  - des particules monodisperses,
  - un milieu anhydre non pulvérulent physiologiquement acceptable permettant la formation sur un support sur lequel la composition est appliquée d'un réseau ordonné des particules monodisperses,

- une deuxième composition cosmétique à appliquer sur le support avant l'application de la première composition, de façon à améliorer l'adhérence de celle-ci sur le support et lisser les surfaces kératiniques,
- une troisième composition cosmétique à appliquer sur la première composition de façon à en changer la couleur ou une autre caractéristique visible et éventuellement améliorer la tenue de la deuxième composition.

Couche de base

La couche de base peut être non anhydre, et comporter par exemple jusqu'à 90 % d'eau relativement au poids total de la composition de cette couche.

La couche de base est compatible avec son application sur les matières kératiniques, par exemple la peau, les lèvres, les ongles, les cils ou les cheveux, selon la nature du maquillage recherché, notamment l'un de ceux énumérés plus haut.

La couche de base peut comporter un polymère choisi notamment parmi les polymères filmogènes.

La couche de base peut, selon différents aspects de l'invention, exercer une ou plusieurs des fonctions suivantes :

- la couche de base peut lisser le support avant l'application de la composition comportant les particules monodisperses afin de faciliter la formation des premières couches du réseau et l'obtention d'un réseau avec des zones monocristallines les plus larges possibles,
- la couche de base peut colorer le support afin de faire ressortir ou modifier la couleur produite par le réseau. A cet effet, la couche de base peut comporter au moins un agent de coloration permettant de diminuer la clarté du support. La couche de base peut par exemple comporter un pigment ou un colorant noir, ou d'une couleur autre, afin de créer un fond coloré permettant d'ajouter une couleur additionnelle à une couleur donnée par le réseau de particules monodisperses. Parmi les colorants ou pigments pouvant être présents dans la couche de base, on peut citer notamment l'oxyde de fer noir, le noir de carbone, le dioxyde de titane noir ;
- la couche de base peut améliorer l'adhérence de la composition contenant les particules monodisperses sur le support maquillé. A cet effet, la couche de base peut comporter au moins un polymère présentant des propriétés adhésives ou pro-adhésives, c'est-à-dire susceptible de devenir adhésif par interaction avec un autre composé. Le polymère peut notamment présenter des propriétés adhésives ou pro-adhésives au sens donné dans les brevets FR 2834884, FR 2811546 et FR 2811547.

La couche de base peut encore exercer une action sur la tension de surface des matières kératiniques afin de permettre par exemple une bonne mouillabilité par la couche de composition contenant les particules monodisperses et favoriser l'empilement des particules monodisperses.

La couche de base peut comporter un même polymère assurant au moins deux des fonctions précitées, par exemple celles de lissage et d'augmentation de l'adhérence, voire éventuellement une fonction de coloration.

La couche de base peut être formulée en fonction de la nature des particules monodisperses.

Dans des exemples non limitatifs de mise en oeuvre de l'invention, les particules monodisperses peuvent être en polystyrène et la couche de base comporter une dispersion non aqueuse NAD dans l'isododécane ou les polymères DAITOSOL (Daito Kasei) ou ULTRASOL (Ganz Chemical). Dans d'autres exemples, les particules monodisperses étant en silice, la couche de base peut comporter un polymère Eastman AQ (20 %) ou du PVA (10 %).

La couche de base peut comporter une phase volatile.

Le polymère est de préférence apte à former un film après application et séchage de la composition. La formation du film peut se faire avec l'aide d'un agent de coalescence. Le polymère peut être en dispersion ou en solution dans une phase aqueuse ou anhydre. De préférence ce polymère est en dispersion dans l'eau ou dans une huile. De manière encore plus préférentielle le polymère contient au moins une fonction susceptible de s'ioniser en solution aqueuse comme un acide carboxylique. Le polymère sera de préférence non soluble au contact d'une phase aqueuse après application et séchage.

Il est également possible d'utiliser selon ce procédé dans la couche de base des monomères ou prépolymères qui sont aptes aussi à polymériser après application sur la peau, soit par action des UV, de la chaleur ou de la présence d'eau par exemple. On peut citer par exemples les monomères cyanoacrylates ou les polymères silicones de faible masse portant des fonctions réactives.

Comme exemple de polymères en dispersion aqueuse on peut citer : Ultrasol 2075 de la société Ganz Chemical, Daitosol 5000AD de Daito Kasei, Avalure UR 450 de Noveon, DYNAMX de National Starch, Syntran 5760 de Interpolymer, Acusol OP 301 de Rohm&Haas, Neocryl A 1090 de Avecia.

Comme exemple de polymères en dispersion huileuse on peut citer : NAD et polymères tels que divulgués dans la demande de brevet EP-A-1 411 069 de la société L'Oréal ou la dispersion de polymère acrylique-silicone ACRIT 8HV-1023 de la société Tasei Chemical Industries.

La phase volatile peut être une phase aqueuse ou une phase anhydre.

Dans le cas d'une phase aqueuse elle est constituée, de préférence d'eau, d'alcool et de glycol.

Dans le cas d'une phase anhydre elle est constituée de préférence, d'au moins une huile volatile.

Couche de recouvrement

La couche de recouvrement peut notamment avoir pour fonction le changement d'une caractéristique visible telle que la couleur ou la brillance et/ou la fonction d'améliorer la tenue du réseau de particules monodisperses sur le support, notamment d'augmenter la résistance à la friction du réseau et éviter son effritement.

La couche de recouvrement peut comporter un ou plusieurs polymères susceptibles de pénétrer ou non dans le réseau de particules, la pénétration du polymère pouvant amener un changement de l'indice de réfraction du milieu autour des particules et donc un changement de la couleur, comme évoqué plus haut.

La couche de recouvrement peut présenter une phase volatile, ce qui peut permettre de limiter dans le temps le changement de couleur, celui-ci pouvant cesser lors de l'évaporation de la phase volatile.

La deuxième composition peut notamment comporter une huile volatile, telle que définie ci-dessus.

La couche de recouvrement peut comporter un solvant non volatil, ce qui peut accroître la durabilité du changement de couleur. Ce solvant va pénétrer et rester dans le milieu entre les particules et modifier là aussi l'indice de réfraction autour des particules.

La deuxième composition destinée à former la couche de recouvrement peut ainsi comporter une huile non volatile, telle que définie ci-dessus.

La couche de recouvrement peut avoir une transparence élevée pour éviter d'affecter la couleur et/ou l'intensité de la couleur provenant du réseau de particules monodisperses.

La couche de recouvrement peut encore être colorée afin par exemple d'exercer une influence sur la couleur et/ou la brillance produite par le réseau de particules monodisperses.

La couche de recouvrement peut encore ralentir la prise d'humidité ou le séchage de la couche de composition contenant le réseau ordonné et réduire la variabilité du résultat au cours du temps.

La couche de recouvrement peut encore, au contraire, accroître la sensibilité à l'environnement, afin de créer par exemple une dépendance de la couleur à la température ou à l'humidité ambiante.

La couche de recouvrement comporte de préférence un polymère filmogène.

La formulation de la couche de recouvrement peut être adaptée à la nature des particules monodisperses.

Dans l'exemple de particules monodisperses en silice ou en polystyrène, la couche de revêtement peut comporter une dispersion non aqueuse NAD dans l'isododécane. Lorsque les particules monodisperses sont en polystyrène, la couche de revêtement peut comporter par exemple un copolymère acrylique ou du PVA. Pour des particules monodisperses en polystyrène, la couche de recouvrement comporte par exemple une dispersion non aqueuse NAD, du PVA (10 %) ou les polymères Eastman AQ (20 %), DAITOSOL ou ULTRASOL.

La couche de recouvrement peut contenir des particules monodisperses ayant une taille moyenne différente de celles des particules monodisperses recouvertes par la couche de recouvrement. Cela peut permettre de changer la couleur de la composition sous-jacente. La couche de recouvrement peut dans ce cas être recouverte, éventuellement, par une couche destinée à en améliorer la tenue.

### Additifs

La composition cosmétique contenant les particules monodisperses, la couche de base et la couche de recouvrement peuvent comporter au moins un additif choisi parmi les adjuvants habituels dans le domaine cosmétique, tels que les charges, les gélifiants lipophiles, les actifs liposolubles, les conservateurs, les hydratants tels que les polyols et notamment la glycérine, les séquestrants, les antioxydants, les solvants, les parfums, les filtres solaires physiques et chimiques, notamment aux UVA et/ou aux UVB, les absorbeurs d'odeur, les ajusteurs de pH (acides ou bases) et leurs mélanges.

Le ou les additifs peuvent notamment être choisis parmi ceux cités dans le CTFA Cosmetic Ingredient Handsbook, 10ème Edition Cosmetic and fragrance Assn, Inc., Washington DC (2004), incorporé ici par référence.

### Formes galéniques

La composition contenant les particules monodisperses peut se présenter sous différentes formes galéniques utilisées dans le domaine cosmétique, utilisées pour une application topique : gels, crèmes, solutions, suspensions, lotions.

La composition peut se présenter sous forme de solution huileuse, notamment gélifiée.

La composition selon l'invention contenant les particules monodisperses peut constituer une composition de soin, de maquillage et/ou de protection solaire. Dans le cas d'une composition de protection solaire, la taille des particules pourra être choisie afin de réfléchir les longueurs d'ondes des UVA et/ou UVB, le choix de la taille des particules s'effectuant par exemple à partir de la relation de Bragg $m\lambda=2nds\sin\theta$, où m est l'ordre de diffraction, n l'indice de réfraction moyen du milieu, $\theta$ l'angle de l'incidence entre la lumière incidente et de la distance entre les plans de diffraction.

La composition peut se présenter sous la forme d'un produit de maquillage du visage, notamment de la peau et/ou des lèvres, des yeux ou des ongles.

### Procédés de maquillage

L'invention a encore pour objet un procédé de maquillage des matières kératiniques, comportant les étapes suivantes :

- appliquer sur un support à maquiller une couche de base,
- appliquer sur la couche de base une composition cosmétique comportant des particules monodisperses et un milieu anhydre non pulvérulent permettant la formation d'un réseau ordonné des particules monodisperses.

Un tel procédé permet d'améliorer la qualité d'application de la composition comportant les particules monodisperses et permet également d'obtenir une bonne « cristallisation » après application sur la peau ou les cheveux par exemple.

La couche de base permet, comme mentionné plus haut, de contrôler et d'uniformiser les propriétés de surface des matières kératiniques, notamment la tension de surface. Elle permet également de lisser et d'uniformiser les rugosités de surface.

En dehors de l'amélioration très sensible de l'arrangement des particules la couche de base peut éventuellement avoir pour effet de fixer la couche de particules monodisperses, la rendant plus stable vis-à-vis des agressions extérieures.

La couche de base, selon ce procédé, contient de préférence un polymère et une phase volatile.

La couche de base peut comporter, comme mentionné précédemment, un polymère ayant des propriétés adhésives et/ou un agent de coloration, notamment de couleur noire.

L'application de la composition contenant les particules monodisperses peut s'effectuer après séchage de la couche de base, par exemple pendant une durée supérieure ou égale à 30 s.

L'invention a encore pour objet, selon un autre de ses aspects, un procédé comportant les étapes suivantes :

- appliquer sur un support à maquiller, éventuellement recouvert d'une couche de base, une composition comportant des particules monodisperses et un milieu permettant la formation d'un réseau ordonné de particules monodisperses,
- appliquer sur le dépôt de la composition contenant les particules monodisperses une couche de recouvrement permettant d'améliorer la tenue de la couche de composition contenant les particules monodisperses.

La couche de recouvrement peut comporter un polymère filmogène.

L'application de la couche de recouvrement peut s'effectuer après séchage de la couche de composition contenant les particules monodisperses, par exemple pendant une durée supérieure ou égale à 30 s.

L'invention a encore pour objet un procédé dans lequel on forme un premier réseau de particules monodisperses ayant une taille moyenne, puis on forme au-dessus de ce premier réseau un deuxième réseau de particules monodisperses ayant une taille moyenne différente de celles du premier réseau.

L'invention a encore pour objet, selon un autre de ses aspects, un procédé comportant les étapes suivantes :

- appliquer une première composition comportant des particules monodisperses et un milieu permettant la formation d'un réseau de ces particules,
- appliquer sur la première composition une deuxième composition permettant de changer la couleur ou une autre caractéristique visible de la première composition, notamment en modifiant l'indice de réfraction du milieu autour du réseau de particules et/ou en modifiant la distance entre les particules du réseau.

La demanderesse a notamment trouvé qu'il est possible de modifier à façon la coloration obtenue par une première composition cosmétique par une deuxième composition non colorée, appliquée ultérieurement.

Le réseau cristallin formé par la première composition peut être composé d'une couche continue ou bien d'îlôts discontinus. La lumière est diffractée par ce réseau cristallin et la longueur d'onde diffractée dépend de la distance entre les particules et de l'indice de réfraction.

La deuxième composition, qui forme la couche de recouvrement, peut contenir au moins un milieu liquide susceptible de pénétrer dans la première composition et de modifier la distance entre les particules et/ou l'indice de réfraction. Le milieu liquide peut être volatile ou non. Dans le cas ou il est entièrement volatil, le changement de couleur est temporaire et la couleur revient progressivement à l'état initial. Dans le cas où une proportion importante du milieu liquide est non volatile on peut obtenir un changement durable de la couleur.

Le réseau cristallin peut être compact ou non, continu ou non. Il peut être formé préalablement à l'application ou se former pendant l'application.

La deuxième composition peut contenir au moins une phase liquide, laquelle peut venir gonfler le réseau ou modifier l'indice de réfraction du milieu. Dans le cas d'un simple changement d'indice de réfraction, la phase liquide pourra avoir un indice de réfraction différent du milieu entourant les particules monodisperses.

La deuxième composition peut également contenir un polymère afin de fixer la première composition.

Il est également possible d'utiliser des monomères ou prépolymères qui sont aptes aussi à polymériser après application sur la peau, soit par action des UV, de la chaleur ou de la présence d'eau par exemple. On peut citer par exemples les monomères cyanoacrylates ou les polymères silicones de faible masse portant des fonctions réactives.

Une couche de base colorée ou non colorée peut éventuellement être appliquée avant ces deux compositions sur les matières kératiniques.

L'invention a encore pour objet, selon un autre de ses aspects, un procédé dans lequel on forme un réseau de particules monodisperses sur les matières kératiniques et on applique sur ce réseau une composition permettant de modifier l'indice de réfraction autour des particules du réseau, notamment celles de la couche de surface du réseau, ce qui peut permettre d'en changer la couleur.

**Modes d'application**

La composition contenant les particules monodisperses, ainsi éventuellement que les compositions destinées à former les couches de base et de recouvrement, peut être appliquée en utilisant un applicateur, de préférence floqué, par exemple un embout ou une mousse floquée, ou un pinceau, notamment à poils fins et souples.

L'application peut encore s'effectuer autrement, par exemple au moyen d'une mousse, d'un feutre, d'une spatule, d'un fritté, d'une brosse, d'un peigne, d'un tissé ou non tissé.

L'application peut encore s'effectuer avec le doigt ou en déposant directement la composition sur le support à traiter, par exemple par pulvérisation ou projection à l'aide par exemple d'un dispositif piézoélectrique ou par transfert d'une couche de composition préalablement déposée sur un support intermédiaire.

La composition contenant les particules monodisperses peut être appliquée selon une épaisseur comprise par exemple entre 1 et 10 $\mu$m, mieux entre 2 et 5 $\mu$m.

L'application de la composition contenant les particules monodisperses s'effectue par exemple avec une densité massique comprise entre 1 et 5 mg/cm$^2$.

Le réseau de particules monodisperses qui est formé comporte par exemple au moins six couches de particules, mieux entre six et vingt couches.

L'application de la composition sur les matières kératiniques peut se faire de manière à permettre au réseau de particules monodisperses de se former après le dépôt. Ainsi, le milieu de la composition peut être formulé de telle sorte que l'évaporation du ou des solvants qu'il contient soit suffisamment lente pour laisser le temps aux particules de s'ordonner et pour limiter également le risque d'agglomération désordonnée des particules avant l'application.

La couche de recouvrement est par exemple appliquée sur une épaisseur allant de 0,5 à 10 $\mu$m. La couche de base est par exemple appliquée sur une épaisseur allant de 0,5 à 10 $\mu$m.

L'application de la couche de recouvrement peut s'effectuer par pulvérisation, ce qui permet de réduire le risque d'endommagement du réseau ordonné sous-jacent.

## Conditionnement

La composition peut être conditionnée dans tout réceptacle ou sur tout support prévu à cet effet.

La composition peut se présenter sous la forme d'un kit comprenant deux compositions conditionnées dans deux réceptacles séparés.

La composition peut se présenter sous la forme d'un kit comprenant un premier réceptacle contenant la composition comportant les particules monodisperses et un deuxième réceptacle contenant l'une au moins des compositions destinées à former la couche de base et la couche de recouvrement.

## Exemples

Les proportions indiquées sont massiques, sauf si le contraire est spécifié.

Exemple 1 : Composition pour les lèvres

Dans l'exemple de composition décrit ci-après, on utilise, à titre de composés X et Y, la combinaison des mélanges A et B suivants préparés par la société Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

Des particules de polystyrène (taille 216 nm, CV = 3,5 %, produit Optibind commercialisé par la société Seradyn) sont concentrées par centrifugation jusqu'à obtenir une concentration de 40 % puis introduites dans une cellule carrée de 4 cm de côté et de hauteur 5 mm. Les particules sont cristallisées par sédimentation puis l'eau est complètement évaporée par séchage.

Une composition est ensuite obtenue à partir de :

| | |
|---|---|
| Particules de polystyrène | 35,0 % |
| Mélange A | 32,9 % |
| Mélange B | 32,1 % |

selon le mode opératoire suivant.

Le mélange des mélanges A et B est introduit au contact des particules de polystyrène puis on laisse réagir pendant 18 heures à 60 °C.

L'excès d'élastomère de silicone au-dessus des particules est ensuite retiré. Cette dernière étape est répétée encore une fois.

La formulation résultante est de couleur verte et peut s'appliquer sans sécher sur les lèvres.

Exemple 2 : Composition de fard à paupière

La composition a la formulation suivante :

| | |
|---|---|
| Particules de polystyrène | 30 % |
| Pentaérythrityl tetraisostéarate | 67,5 % |
| PEG Dimethicone | 2,5 % |

Cette composition peut être préparée de la façon suivante.

Les particules de polystyrène, initialement à une concentration de 10 % sont d'abord concentrées en solution aqueuse jusqu'à une concentration de 40 %. L'huile et le tensioactif siliconé sont mélangés séparément et ajoutés sous faible agitation pour former une émulsion huile dans eau. L'eau est ensuite progressivement complètement évaporée.

Une couche de base peut être appliquée sur les paupières avant la composition ci-dessus.

Une coloration bleue-verte est obtenue après application de la composition.

Couche de base

La couche de base a la formulation suivante :

| | |
|---|---|
| Ultrasol® 2075 (Ganz Chemical)* | 80 % |
| Cab-O-Jet 200 Black Colorant** | 10 % |
| Eau | 10 % |
| * Copolymère d'Acrylate/ammonium méthacrylate en dispersion dans l'eau à une concentration massique de 50 %. ** Noir de carbone de taille 130 nm en dispersion aqueuse à 20 % commercialisé par la société Cabot Corp. | |

Bien entendu, l'invention n'est pas limitée aux exemples décrits. D'autres agents de coloration peuvent notamment être ajoutés.

L'expression « comportant un » doit être comprise comme étant synonyme de « comportant au moins un », sauf si le contraire est spécifié.

L'expression « compris entre » doit s'entendre bornes incluses, sauf si le contraire est spécifié.

**Revendications**

**1.** Composition cosmétique comportant :

- un milieu physiologiquement acceptable, anhydre et non pulvérulent, comportant une phase huileuse,
- des particules monodisperses aptes à former un réseau ordonné de particules monodisperses sur un support sur lequel la composition est appliquée.

**2.** Composition selon la revendication précédente, le milieu comportant au moins une huile volatile.

**3.** Composition selon l'une des revendications 1 à 2, comportant au moins 15 % en poids de particules monodisperses, par rapport au poids total de la composition.

**4.** Composition selon l'une quelconque des revendications précédentes, la teneur massique en particules monodisperses étant supérieure ou égale à 20 % en poids, notamment strictement supérieure à 20 % en poids par rapport au poids total de la composition.

**5.** Composition selon la revendication 4, la teneur massique en particules monodisperses étant supérieure ou égale à 25 % en poids par rapport au poids total de la composition.

**6.** Composition selon l'une quelconque des revendications 1 à 5, dans laquelle le rapport en poids de la phase huileuse sur le poids total de particules présentes dans la composition est supérieur à 0,5.

**7.** Composition selon la revendication 6, dans laquelle ledit rapport est supérieur à 0,75.

**8.** Composition selon l'une quelconque des revendications précédentes, dans laquelle les particules monodisperses sont hydrophobes.

**9.** Composition selon l'une quelconque des revendications précédentes, dans laquelle le milieu dérive de la réaction d'hydrosilylation, de condensation ou de réticulation en présence d'un peroxyde d'au moins un composé X et d'au moins un composé Y, l'un au moins des composés X et Y étant siliconé.

**10.** Composition selon la revendication 9, dans laquelle les composés X et Y sont des polymères siliconés, l'un au moins des composés X et Y étant de préférence porteur d'au moins un groupe polaire.

**11.** Procédé de maquillage des matières kératiniques comportant l'application sur celles-ci d'une composition telle que définie dans l'une quelconque des revendications précédentes.

**12.** Kit comportant :

- une première composition à appliquer sur les matières kératiniques,
- une deuxième composition à appliquer sur la première, cette deuxième composition comportant :
- un milieu physiologiquement acceptable, anhydre et non pulvérulent,
- des particules monodisperses aptes à former un réseau ordonné de particules monodisperses sur un support sur lequel la composition est appliquée.

**13.** Kit selon la revendication 12, dans lequel l'une des première et deuxième compositions comprend en outre au moins un composé X, et l'autre des première et deuxième compositions comprend en outre au moins un composé Y, l'un au moins desdits composés étant siliconé, et lesdits composés X et Y étant susceptibles de réagir ensemble par une réaction d'hydrosilylation, de condensation ou de réticulation en présence d'un peroxyde, lorsqu'ils sont mis en contact l'un avec l'autre.

**EP 1 913 933 A1**

Office européen
des brevets

**RAPPORT DE RECHERCHE EUROPEENNE**

Numéro de la demande

EP 07 11 8483

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 945 095 A (MOUGIN NATHALIE [FR] ET AL) 31 août 1999 (1999-08-31) * exemples 1-12 * ----- | 1-13 | INV. A61K8/81 A61Q1/04 A61Q1/10 |
| X | US 2004/180027 A1 (KUMAR MANOJ [US] ET AL) 16 septembre 2004 (2004-09-16) * alinéas [0105], [0176] - [0180], [0182], [0186], [0187], [0189] * ----- | 1-13 | A61K8/04 A61K8/25 A61Q1/00 A61Q1/06 A61Q1/08 |
| X | JP 2003 012461 A (SHISEIDO CO LTD) 15 janvier 2003 (2003-01-15) * revendication 1; tableaux 1-3,5,7 * ----- | 1-13 | |
| X | US 5 658 574 A (BAHARY WILLIAM SHAUL [US] ET AL) 19 août 1997 (1997-08-19) * colonne 12, ligne 59,60; exemple 71 * ----- | 1-13 | |

**DOMAINES TECHNIQUES RECHERCHES (IPC)**

A61K

Le présent rapport a été établi pour toutes les revendications

| Lieu de la recherche | Date d'achèvement de la recherche | Examinateur |
|---|---|---|
| Munich | 11 février 2008 | Vayssié, Stéphane |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................................
& : membre de la même famille, document correspondant

EPO FORM 1503 03.82 (P04C02)

24

**ANNEXE AU RAPPORT DE RECHERCHE EUROPEENNE
RELATIF A LA DEMANDE DE BREVET EUROPEEN NO.**

EP 07 11 8483

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-02-2008

| Document brevet cité au rapport de recherche | | Date de publication | Membre(s) de la famille de brevet(s) | | Date de publication |
|---|---|---|---|---|---|
| US 5945095 | A | 31-08-1999 | AT | 157529 T | 15-09-1997 |
| | | | CA | 2197498 A1 | 09-01-1997 |
| | | | DE | 69600059 D1 | 09-10-1997 |
| | | | DE | 69600059 T2 | 05-02-1998 |
| | | | DK | 749746 T3 | 23-02-1998 |
| | | | EP | 0749746 A1 | 27-12-1996 |
| | | | ES | 2110857 T3 | 16-02-1998 |
| | | | WO | 9700662 A1 | 09-01-1997 |
| | | | GR | 3025474 T3 | 27-02-1998 |
| | | | JP | 3027008 B2 | 27-03-2000 |
| | | | JP | 10502389 T | 03-03-1998 |
| | | | KR | 100231637 B1 | 15-11-1999 |
| US 2004180027 | A1 | 16-09-2004 | US | 2005142094 A1 | 30-06-2005 |
| JP 2003012461 | A | 15-01-2003 | AUCUN | | |
| US 5658574 | A | 19-08-1997 | AU | 698631 B2 | 05-11-1998 |
| | | | AU | 7216896 A | 07-05-1997 |
| | | | BR | 9611098 A | 13-07-1999 |
| | | | DE | 69618973 D1 | 14-03-2002 |
| | | | DE | 69618973 T2 | 25-07-2002 |
| | | | WO | 9714404 A1 | 24-04-1997 |
| | | | EP | 0854704 A1 | 29-07-1998 |
| | | | ES | 2170872 T3 | 16-08-2002 |
| | | | ZA | 9608476 A | 08-04-1998 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0047167 A
- WO 02056854 A
- WO 05018566 A
- WO 2006097332 A
- WO 05012961 A
- FR 0302809
- JP 2001011340 A
- WO 9936477 A
- US 6299979 B
- US 6387498 B
- US 5825643 A
- WO 9619347 A
- WO 9936478 A

- US 6818051 B
- US 6894086 B
- EP 1634619 A
- WO 04055081 A
- FR 2232303 A
- US 5162410 A
- WO 2004073626 A
- EP 1411069 A
- WO 04028488 A
- WO 0196450 A
- FR 2834884
- FR 2811546
- FR 2811547

**Littérature non-brevet citée dans la description**

- **XIA et al.** *Adv. Mater,* 2000, vol. 12, 693-713
- **ISHIZU et al.** *Kagaku To Kogyo,* 2004, vol. 57 (7
- **OKUBO et al.** *Colloid & Polymer Science,* 2002, vol. 280 (3), 290-295

- **XU et al.** *Chem. Mater.,* 2002, vol. 14, 1249-1256
- **H. FUDOUZI et al.** *Langmuir,* 2003, vol. 19, 9653-9660
- CTFA Cosmetic Ingredient Handsbook. Cosmetic and fragrance Assn, Inc, 2004